# EUROPEAN PATENT APPLICATION

(11) **EP 1 649 883 A1**
(43) Date of publication of application: **26.04.2006**
(21) Application number: 05100749.0
(22) Date of filing: 03.02.2005
(51) Int. Cl.: A61M 1/16

(54) **A CO2 removal device for removing carbon dioxide from blood or from a fluid taken from patient's cardio-circulatory system**

(30) Priority: 19.10.2004 EP 04425786
(71) Applicant: MRI S.r.l. Società Unipersonale, 45100 ROVIGO (IT)
(72) Inventor: FORINO, Luciano, 10100, TORINO (IT)
(74) Representative: Jorio, Paolo

(57) **Abstract**

A CO₂ removal device (1) for removing carbon dioxide from the blood is described, provided with a first inlet (7) through which it receives the blood to be treated, with an outlet (13) for the treated blood and with a second inlet (9) for feeding the oxygen with which the blood is to be treated. The CO₂ removal device (1) comprises measuring means (21) for continuously measuring characteristic parameters of the treated blood.

## Description

The present invention related to a CO₂ removal device for removing carbon dioxide from the blood or from a fluid taken from patient's cardio-circulatory system.

In particular, known CO₂ removal devices are used on patients in critical conditions which need to accompany an integrative respiratory therapy to an extra-renal therapy able to perform the functions normally carried out by healthy kidneys, purifying the patient's blood.

In greater detail, known CO₂ removal devices are connected to the patient's cardio-circulatory system through a pair of conduits for transporting the blood, or catheters, whereof one receives the blood to be treated from an artery or vein of the patient and makes it available at the inlet of the device itself, whilst the other one is able to be introduced into an artery or into a vein of the patient to re-inject the treated blood exiting the aforesaid device into the patient's cardio-circulatory system.

In particular, known CO₂ removal devices essentially comprise a case provided with a first inlet connected to a conduit for receiving the blood to be treated, a second inlet connectable to an oxygen tank from which it receives a predetermined quantity of oxygen required to oxygenate the blood, a first discharge outlet through which the carbon dioxide removed from the blood undergoing treatment is expelled, and lastly a second outlet through which is supplied the treated blood whereto oxygen was added and wherefrom carbon dioxide was removed.

More in detail, the aforesaid case houses a bundle of hollow fibres or capillary tubes which, in use, are traversed by the oxygen coming from the second inlet and are lapped by the blood undergoing treated which comes from the first inlet, in such a way as to allow the haemoglobin of the blood to release carbon dioxide and simultaneously acquire oxygen.

Moreover, the treated blood moves away from the case through the second outlet, whilst the carbon dioxide is discharged from the case through the first outlet.

In particular, in known CO₂ removal devices, the evaluation of the effectiveness of the ongoing therapy is based on a periodic measurement of the concentration percentage of carbon dioxide in the treated blood, obtained through withdrawals from the patient's arteries.

In particular, this measurement system is invasive and requires relatively long times for analysing the sample, i.e. about 15-20 minutes, to make available the information about the concentration percentage of carbon dioxide in the blood.

Consequently, an inevitable delay is generated between the time when the blood sample is obtained and the time when the outcome of the tests on the sample is available to personnel responsible for the therapy.

The aforesaid conditions are sub-optimal for patients in extremely critical conditions and such as to require a substitutive respiratory therapy.

The object of the present invention is to provide a CO₂ removal device, which is free of the drawbacks connected with the known devices specified above, and which allows to eliminate the need for invasive periodic withdrawals and to measure the profile of carbon dioxide concentration in the patient's blood over time in continuous fashion.

The aforesaid object is achieved by the present invention, in that it relates to a CO₂ removal device for removing carbon dioxide from blood as claim in claim 1.

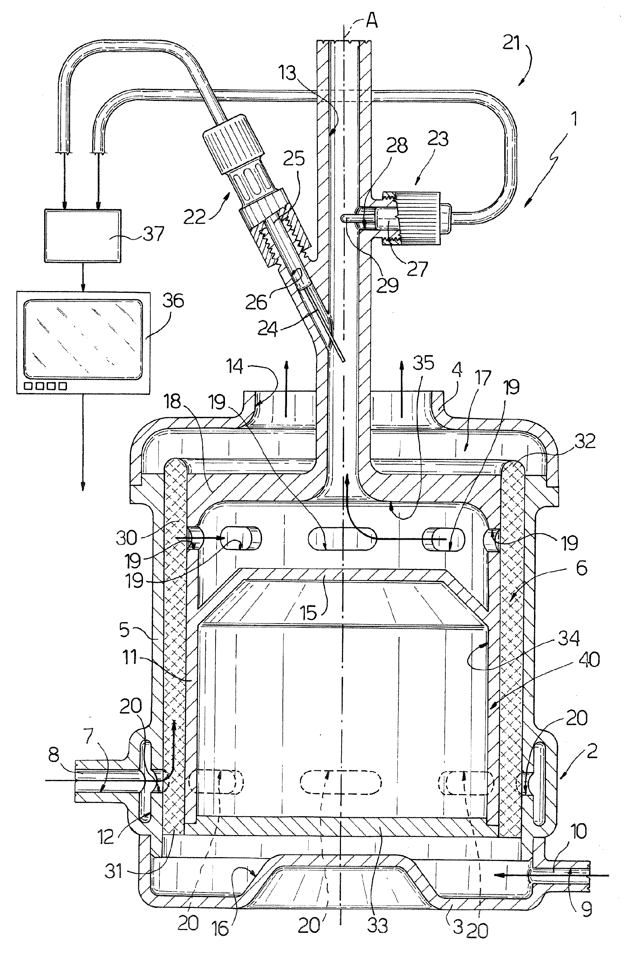

For a better understanding of the present invention, a preferred embodiment shall now be described of a CO₂ removal device for removing carbon dioxide from blood, purely by way of non limiting example and with reference to the accompanying drawing which shows a section view thereof.

With reference to the accompanying figure, the reference number 1 globally designates a CO₂ removal device essentially comprising a hollow case 2, substantially cylindrical, with axis A and traversed in use by the blood to be treated, and an oxygen/carbon dioxide exchanger 6, constituted in the illustrated case by a bundle of fibres with tubular shape and housed within the case 2 for the removal of carbon dioxide from the blood being treated by oxygenation of the blood.

In particular, the case 2 has opposite axial end portions 3,4 with prevalently discoidal shape, and a substantially cylindrical intermediate portion 5.

In greater detail, the end portion 3 defines a radial inlet seat 9 for a conduit 10 for feeding the pressurised oxygen with which the patient's blood is to be treated.

The end portion 4 axially defines an outlet seat 14 for discharging the carbon dioxide removed from the treated blood.

The intermediate portion 5 fully houses the exchanger 6 and defines a radial inlet seat 7, positioned in proximity to the end portion 3 and at the diametrically opposite side of the seat 9 relative to the axis A, and connectable to a conduit or catheter 8 for the adduction of the blood to be treated which comes from the patient's cardio-circulatory system.

The intermediate portion 5 also defines an annular chamber 12 connected in a position radially more external to the conduit 8 and communicating with the space in which is housed the exchanger 6 by means of a plurality of through opening 20 to allow the flow of the blood towards the exchanger 6.

The exchanger 6 is formed by a cylindrical tubular element 30, whose opposite axial ends 31,32 respectively face the end portions 3 and 4 of the case 2.

In greater detail, the tubular element 30 can be constituted by a plurality of sheets of hollow fibres made of polymeric material and possibly imbued with an anti-coagulating agents such as heparin.

The tubular element 30 is sandwiched between the intermediate portion 5 of the case 2 and a tubular body 40 with axis A positioned radially more internal.

As indicated by the arrow in the accompanying figure, the tubular element 30 of the exchanger 6 receives the blood to be treated by means of the through holes 20 obtained along the inner circumferential surface of the intermediate portion 5 and releases the treated blood by means of a plurality of through openings 19 obtained through the aforesaid body 40.

In particular, the body 40 integrally comprises a first axial portion 11 co-operating with the exchanger 6 at the opposite side of the intermediate portion 5 of the case 2 and bearing the openings 19, a second axial portion having smaller section than the axial portion 11 and defining a conduit 13 for the outflow of the treated blood, and a radial portion 18 joining the axial portion to each other; the body 40 is also provided with a plate 33 mounted on the free end of the axial portion 11 in such a way as to close the body 40 from the side oriented towards the end portion 3 of the case 2.

More precisely, the conduit 13 extends through the seat 14 of the case 2, projects in overhang from the case 2 and is able to be connected, e.g. through a catheter, to the patient's cardio-circulatory system to re-injected the treated blood into it.

In particular, a bell element 15 extends integrally from the inner circumferential surface of the axial portion 11, in axially intermediate position between the plurality of openings 19 and 20 thereby subdividing the body 40 in two compartments 35, 34 oriented respectively towards the end portions 4 and 3 of the case 2.

In detail, the compartment 35 is axially delimited by the bell element 15 and by the radial portion 18 and radially delimited by the segment of the axial portion 11 which is between the bell element 15 and the radial portion 18; the compartment 35 communicates outwards through the openings 19 and the conduit 13.

The compartment 34 is axially delimited by the bell element 15 and by the plate and radially delimited by the segment of the axial portion 11 which is between the bell element 15 and the plate 33; the compartment 34 communicates outwards solely through the openings 20.

The case 2 and the body 40 delimit between them a pair of interspaces 16, 17, for collecting respectively the oxygen flowing in through the seat 9 and the carbon dioxide flowing out from the exchanger 6.

In detail, the interspace 16 is essentially defined between the end portion 3 of the case 2 and the plate 33 and it is placed in communication with the conduit 10 for feeding the oxygen through the seat 9. In similar manner, the interspace 17 is essentially defined between the end portion 4 of the case 2 and the radial portion 18 of the body 40 and it is placed in communication, through the seat 14, with an environment for discharging the carbon dioxide removed from the blood undergoing treatment.

According to an important aspect of the present invention, the CO₂ removal device 1 is provided with sensing means 21 for continuously measuring characteristic parameters of the treated blood, in particular carbon dioxide concentration and temperature.

In greater detail, the aforesaid sensing means 21 comprise a fibre optics photometric sensor 22 and a thermocouple 23, whose measurements are shown by means of a display 36.

More in particular, the photometric sensor 22 comprises a support body 25 housed inside a first branch conduit 26 of the conduit 13, and a sensitive fibre optics element 24 projecting in overhang from the support body 25, extending in part in the conduit 13 and placed in continuous contact with the treated blood. Moreover, the support body 25 is electrically connected to a data processing unit 37 and to the display 36.

The thermocouple comprises support body 27 housed inside a second branch conduit 28 of the conduit 13, and a sensitive element 29 projecting in overhang from the support body 27, extending in part in the conduit 13 and placed in continuous contact with the treated blood to measure the temperature thereof. Moreover, the support body 27 is electrically connected to the data processing unit 37 and to the display 36.

In use, the blood to be treated under pressure enters the CO₂ removal device 1 through the seat 7, flows in the annular chamber 12 and therefrom inside the tubular element 30 of the exchanger 6 by means of the through openings 20.

At the same time, the oxygen under pressure enters through the seat 9 into the CO₂ removal device and, through the interspace 16, it accesses the axial end 31 of the exchanger 6.

The tubular element 30 of the exchanger 6 is traversed by the flow of blood and simultaneously by the oxygen under pressure.

During the circulation of the blood undergoing treatment and of the oxygen through the exchanger 6, the haemoglobin of the blood acquires the oxygen and releases the excess carbon dioxide, which leaves the exchanger 6 through the axial end 32, traverses the interspace 17 and exits by means of the outlet seat 14.

The treated blood is conveyed through the openings 19 from the exchanger 6 in the compartment 35, it accesses the conduit 13 and, during the subsequent conveyance towards the catheter connected to the patient's cardio-circulatory system, it laps the sensitive elements 24 and 29 of the photometric sensor 22 and of the thermocouple 23.

The photometric sensor 22, according to the colour of the treated blood that laps the sensitive fibre optics element 24, sends and electrical signal to the data processing unit 37; said signal is correlated to the concentration of carbon dioxide present in the treated blood. In particular, a light/dark colour of the treated blood indicates a low/high concentration of carbon dioxide.

The data processing unit 37 determines, on the basis of the concentration of carbon dioxide in the blood and by means of known relationships, the value of the oxygen concentration, the pH of the blood, base excess, carbonic acid bicarbonate. These values are continuously shown on the display 36.

In wholly similar fashion, the thermocouple 23 sends an electrical signal to the data processing unit 37 correlated to the temperature of the treated blood measured by means of the sensitive element 29. The measured value is continuously shown on the display 36.

From an examination of the characteristics of the CO₂ removal device 1 according to the present invention, the advantages it allows to achieve are readily apparent.

In particular, the CO₂ removal device 1 allows to evaluate in real time the effectiveness of the ongoing therapy, continuously showing the characteristics parameters of the treated blood on the display 36.

Moreover, the measurement of the percent concentration of carbon dioxide in blood and of other characteristic parameters takes place with no need for invasive procedures, such as the taking of blood samples from the patient.

It is readily apparent that the CO₂ removal device 1 described and illustrated herein can be subject to modifications and variants without thereby departing from the scope of protection of the claims.

In particular, the photometric sensor 22 and the thermocouple 23 could be mounted through the end portion 4 of the case 2 and the radial portion 18 of the body 40 thereby placing the respective sensitive elements 24, 29 in projecting position inside the compartment 35 and in continuous contact with the flow of treated blood flowing out from the CO₂ removal device 1.

Moreover, along the flow of the treated blood out of the CO₂ removal device 1 could be provided appropriate reactants to perform the continuous monitoring of the value of the glycemia/insulin ratio.

Lastly, according to a possible not shown alternative, CO₂ removal device 1 could be fed with other types of fluids taken from patient's cardio-circulatory system, such as plasmatic water, blood plasma and other blood substances, which are then infused back into the patient in a non-invasive way after the treatment of CO₂ removal and monitoring of vital parameters integrated in the present invention.

## Claims

1. CO₂ removal device (1) for removing carbon dioxide from patient's blood, provided with a first inlet (7) through which it receives a fluid taken from patient's cardio-circulatory system, with an outlet (13) for the treated fluid and with a second inlet (9) for feeding the oxygen with which said fluid is to be treated, **characterised in that** it comprises sensing means (21) for the continuous, non invasive measurement of characteristic parameters of the treated fluid correlated with characteristic parameters of the treated blood.

2. Device as claimed in claim 1, **characterised in that** said means sensing means (21) comprise measuring means (22,23) interacting with the treated fluid and means (36) for displaying the characteristic parameters of the blood, measured by said measuring means (22,23).

3. Device as claimed in claim 2, **characterised in that** it comprises a case (2) provided with said first and second inlet (7, 9) and with said outlet (13), housing means for treating said fluid (6) and bearing said measuring means (22,23).

4. Device as claimed in any of the previous claims, **characterised in that** said measuring means (22,23) comprise means for determining (22) the concentration of carbon dioxide in the treated blood.

5. Device as claimed in any of the previous claims, **characterised in that** said means for determining (22) the concentration of carbon dioxide are photometric means.

6. Device as claimed in any of the previous claims, **characterised in that** said measuring means (22,23) comprise means for determining (23) the temperature of the treated blood.

7. Device as claimed in claim 6, **characterised in that** said means for determining (23) the temperature are thermoelectric means.

8. Device as claimed in any of the previous claims, **characterised in that** said fluid is blood.

9. Device as claimed in any of the previous claims, **characterised in that** said fluid is plasmatic water.
